# EUROPÄISCHE PATENTSCHRIFT

(11) **EP 0 518 192 B1**
(45) Veröffentlichungstag und Bekanntmachung des Hinweises auf die Patenterteilung: **08.03.1995**
(21) Anmeldenummer: 92109325.8
(22) Anmeldetag: 03.06.1992
(51) Int. Cl.: A61K 7/48, A61K 7/06

(54) **Naturkosmetisches Haut- oder Haarpflegemittel**
Skin and haircare composition containing natural cosmetic products
Composition pour les soins de la peau et des cheveux à base de produits cosmétiques naturels

(30) Priorität: 03.06.1991 DE 4118098
(43) Veröffentlichungstag der Anmeldung: 16.12.1992
(73) Patentinhaber: EXTERNA-JUNIKA KOSMETIKVERTRIEBS-GmbH, D-70178 Stuttgart (DE)
(72) Erfinder: Kalev, Iwan, A-1050 Wien (AT)
(74) Vertreter: Wolff, Michael, Dipl.-Phys.

(56) Entgegenhaltungen:
- FR-A- 2 295 735
- GB-A- 2 228 411
- SEIFEN-ÖLE-FETTE-WACHSE, Band 108, Nr. 13, August 1982, Seiten 402-403,Augsburg, DE; "Neue Produkte auf dem Markt"
- HOUSEHOLD & PERSONAL PRODUCTS INDUSTRY, Band 9, Nr. 8, August 1972, Seiten 42-43; S. Epstein: "Naturals in personal care products"

## Beschreibung

Die Erfindung betrifft ein naturkosmetisches Haut- oder Haarpflegemittel mit einem Gehalt an Säften gepreßter Pflanzen oder -teile, einschließlich Zitronensaft und Olivenöl, und aus Wasser. Es soll äußerlich angewandt werden.
In der französischen Gebrauchsmusterschrift 2.416.010 (78.03251) wird ein derartiges Mittel der biologischen Kosmetik spezifiziert, welches grundsätzlich 5 bis 25 g, insbesondere 20 g/l Zitronensaft, 3 bis 10 g, insbesondere 10 g/l Olivenöl sowie 200 bis 350 g (destilliertes) Wasser enthält und der Verschönerung der Kopfhaare, der Behandlung fetter Haare, der Beendigung ihres Ausfalls, dem Verschwinden von Kopfschuppen und insbesondere dem Nachwachsen der Haare dien-t.
Das erfindungsgemäße Haut- oder Haarpflegemittel zeichnet sich laut Anspruch 1 durch einen Gehalt an Petersiliensaft, Selleriesaft aus Blättern und Knollen, Löwenzahnsaft aus Blättern, Saft geschälter Kiwis, Joghurt, Saft entkernter grüner Oliven und Meersalz aus.
Nach dem Großen Rezeptbuch der Haut- und Körperpflegemittel, Heidelberg 1956, von K. Rothemann werden aus Heilpflanzen bzw. dem Meer für die Hautbehandlung die mit ihren Heilanzeigen nachstehend genannten Säfte gewonnen:
Zitronensaft gegen rauhe, rissige Hände und Sommersprossen
Olivenöl, insbesondere fettes, als wichtiger Bestandteil hochwertiger Salben, Cremes, Haut- und Haaröle
Petersiliensaft (der Frischpflanze) gegen Sommersprossen, Geschwüre und als Haarwuchsmittel (Volksheilkunde)
Löwenzahnkrautsaft gegen Hautekzeme und Flechten
Seesalz gegen schlaffe, vorzeitig gealterte Haut und schwach durchblutete Haut anämischer Personen
Apfel roh geschabt innerlich angewandt gegen Ekzeme
Eine bevorzugte Rezeptur des erfindungsgemäßen Mittels, insbesondere zur Hautpflege, ist im Anspruch 2 wiedergegeben.

Zur Haarpflege wird das zuvor genannte Mittel durch Zusatz einer in Anspruch 3 bestimmten Relativmenge Knoblauchsaft geeignet.
Auch das aus der FR-U-2.416.010 bekannte Mittel dient speziell der Haar- und Haarbodenpflege dadurch, daß es 80 bis 150 g Knoblauch, insbesondere 150 g/l fein gehackte Knoblauchzehen enthält.

Im folgenden ist ein praktisches Beispiel einer erprobten Rezeptur angegeben:

| | |
|---|---|
| Zitronensaft | 13,5 l |
| Olivenöl | 0,5 l |
| Wasser | 5 l |
| Petersiliensaft | 16,5 l |
| Sellerieblattsaft | 12 l |
| Sellerieknollensaft | 8 l |
| Löwenzahnblattsaft | 11 l |
| Kiwisaft | 14,5 l |
| Joghurt | 15,6 l |
| grüne - Olivensaft | 12,2 l |
| Meersalz | 7,5 kg, gelöst |
| Summe | 116,3 l = 580 Flaschen à 200 ml |

Hinzu kommen bei Bedarf 350 g eines Konservierungsstoffes.

Vorstehende Rezeptur gilt für ein Hautpflegemittel. Daraus wird durch Zusatz von 20 l Knoblauchsaft ein Haarpflegemittel, das durch Zusatz von Apfelteilen gewinnen kann.
Den wirksamsten Beitrag leisten die Säfte von Zitrone, Petersilie, Kiwi und Knoblauch sowie das Meersalz.

Gegenstand der Erfindung sind auch die verbesserten Mittel gemäß Ansprüchen 4 bis 6.

## Patentansprüche

1. Naturkosmetisches Haut- oder Haarpflegemittel mit einem Gehalt an Säften gepreßter Pflanzen oder -teile , einschließlich Zitronensaft und Olivenöl, und aus Wasser, **gekennzeichnet** durch einen Gehalt an Petersiliensaft, Selleriesaft aus Blättern und Knollen, Löwenzahnsaft aus Blättern, Saft geschälter Kiwis, Joghurt, Saft entkernter grüner Oliven und Meersalz.

2. Mittel nach Anspruch 1, dadurch gekennzeichnet, daß in 1 l enthalten sind:
142 ml Petersiliensaft, 103 ml Selleriesaft aus Blättern,
69 ml Selleriesaft aus Knollen, 95 ml Löwenzahnsaft,
125 ml Kiwisaft, 116 ml Zitronensaft, 134 ml Joghurt,
105 ml Olivensaft, 64 g Meersalz, 4 ml Olivenöl und
43 ml Wasser.

3. Haarpflegemittel nach Anspruch 2, mit einem Gehalt an Knoblauch, **gekennzeichnet** durch Zusatz von 172 ml Knoblauchsaft auf 1 l.

4. Naturkosmetisches Haut- und Haarpflegemittel mit einem Gehalt an Säften gepreßter Pflanzen oder -teile, einschließlich Zitronensaft, **gekennzeichnet** durch einen Gehalt an Petersiliensaft, Saft geschälter Kiwis, Joghurt und Meersalz.

5. Mittel nach Anspruch 4, dadurch gekennzeichnet, daß in ungefähr 1l enthalten sind: 245 ml Petersiliensaft, 215 ml Kiwisaft, 200 ml Zitronensaft, 230 ml Joghurt und 110 g Meersalz.

6. Haarpflegemittel nach Anspruch 5, mit einem Gehalt an Knoblauch, **gekennzeichnet** durch Zusatz von 172 ml Knoblauchsaft auf 1l.

## Claims

1. Natural cosmetic skincare or haircare agent with a content of juices of pressed plants or plant parts, including lemon juice and olive oil, and of water, characterised by a content of parsley juice, celery juice from leaves and tubers, dandelion juice from leaves, juice of peeled kiwifruit, yoghurt, juice of destoned green olives and sea salt.

2. Agent according to claim 1, characterised thereby that contained in one litre are:
142 ml of parsley juice, 103 ml of celery juice from leaves, 69 ml of celery juice from tubers, 95 ml of dandelion juice, 125 ml of kiwifruit juice, 116 ml of lemon juice, 134 ml of yoghurt, 105 ml of olive juice, 65 grams of sea salt, 4 ml of olive oil and 43 ml of water.

3. Haircare agent according to claim 2, comprising a content of garlic, characterised by addition of 172 ml of garlic juice to one litre.

4. Natural cosmetic skincare or haircare agent with a content of juices of pressed plants or plant parts, including lemon juice, characterised by a content of parsley juice, juice of peeled kiwifruit, yoghurt and sea salt.

5. Agent according to claim 4, characterised thereby that contained in approximately one litre are: 245 ml of parsley juice, 215 ml of kiwifruit juice, 200 ml of lemon juice, 230 ml of yoghurt and 110 grams of sea salt.

6. Agent according to claim 5, comprising a content of garlic, characterised by addition of 172 ml of garlic juice to one litre.

## Revendications

1. Composition pour les soins de la peau ou des cheveux à base de produits cosmétiques naturels, avec une teneur en jus de plantes ou de parties de plantes pressées, jus de citron et huile d'olive y compris, et eau, caractérisée par un teneur en jus de persil, jus de céleri extrait des feuilles et tubercules, jus de pissenlit extrait des feuilles, jus de kiwis pelés, yaourts, jus d'olives vertes dénoyautées et sel marin.

2. Composition suivant la revendication 1, caractérisée en ce qu'1 litre comporte:
142 ml de jus de persil, 103 ml de jus de céleri extrait des feuilles, 69 ml de jus de céleri extrait des tubercules, 95 ml de jus de pissenlit, 125 ml de jus de kiwis, 116 ml de jus de citron, 134 ml de yaourt, 105 ml de jus d'olives, 64 g de sel marin, 4 ml d'huile d'olives et 43 ml d'eau.

3. Composition pour les soins des cheveux suivant la revendication 2, avec une teneur en ail, caractérisée par l'addition de 172 ml de jus d'ail pour 1 l.

4. Composition pour les soins de la peau et des cheveux à base de produits cosmétiques naturels, avec une teneur en jus de plantes ou parties de plantes pressées, jus de citron y compris, caractérisée par une teneur en jus de persil, jus de kiwis pelés, yaourt et sel marin.

5. Composition suivant la revendication 4, caractérisée en ce qu'1 l à peu près contient: 245 ml de jus de persil, 215 ml de jus de kiwis, 200 ml de jus de citron, 230 ml de yaourt et 110 g de sel marin.

6. Composition pour les soins des cheveux suivant la revendication 5, avec une teneur en ail, caractérisée par l'addition de 172 ml de jus d'ail pour 1 l.
